# EUROPEAN PATENT APPLICATION

(11) **EP 4 461 805 A1**
(43) Date of publication of application: **13.11.2024**
(21) Application number: 23737319.6
(22) Date of filing: 06.01.2023
(51) Int. Cl.: C12N 5/073, C12N 5/074, C12N 5/0793

(54) **METHOD FOR CULTURING LIVER-DERIVED CELLS AND CULTURE SYSTEM INCLUDING LIVER-DERIVED CELLS**

(30) Priority: 07.01.2022 JP 2022001828
(71) Applicant: Integriculture Inc., Tokyo 113-0033 (JP)
(72) Inventor: KAWASHIMA, Ikko, Tokyo 113-0033 (JP); KANAYAMA, Takanori, Tokyo 113-0033 (JP); SHINTANI, Keisuke, Tokyo 113-0033 (JP); NAKANE, Chie, Tokyo 113-0033 (JP); KOKIDO, Ibuki, Tokyo 113-0033 (JP); ICHIYAMA, Koji, Tokyo 113-0033 (JP); OCHIAI, Toshiro, Tokyo 113-0033 (JP); KUNIMASA, Kazuhiro, Tokyo 113-0033 (JP)
(74) Representative: Grünecker Patent- und Rechtsanwälte PartG mbB
(86) International application number: PCT/JP2023/000237
(87) International publication number: WO 2023/132371

(57) **Abstract**

The present invention is intended to provide a novel culture method of liver-derived cells. In other words, liver-derived cells are co-cultured with one or more types of cells selected from a group consisting of intestin-derived cells, lung-derived cells, and embryonic membrane-derived cells. Alternatively, the liver-derived cells may be cultured in the presence of the culture supernatant obtained by culturing one or more types of cells selected from the group consisting of intestinal-derived cells, lung-derived cells, and embryonic membrane-derived cells.

## Description

### [Technical Field]

This invention relates to a culture method of liver-derived cells and a culture system containing liver-derived cells.

### [Related Art]

Conventionally, it has beben known that hepatocytes are cultured with feeder cells manufactured from fibroblasts, endothelial cells, epithelial cells, etc. (JP2009-183299A; JP2010-148386A; JP2007-516706A), whereby the hepatocytes form spheroids (JP2010-148386A), and feeder cells provide hepatocytes with intracellular matrix and diffusible factors such as growth factors for proliferation and expansion of hepatocytes.

### [Summary of the invention]

### [Problem to be solved by the invention]

It is an object of this invention to provide a culture method of liver-derived cells and a culture system containing liver-derived cells.

### [Summary of the Invention]

One aspect of the present invention is a method for culturing liver-derived cells, comprising co-culturing the liver-derived cells with one or more types of cells selected from a group consisting of intestine-derived cells, lung-derived cells, and embryonic membrane-derived cells. Head-derived cells and/or kidney-derived cells may also be co-cultured.

Another aspect of the present invention is a method for culturing liver-derived cells, comprising culturing the liver-derived cells in the presence of a culture supernatant obtained by culturing one or more types of cells selected from the group consisting of intestine-derived cells, lung-derived cells, and embryonic membrane-derived cells. The above-mentioned culture supernatant may comprise culture supernatant obtained by culturing the one or more types of cells further with head-derived cells and/or kidney-derived cells. The above-mentioned culture supernatant may comprise culture supernatant obtained by culturing one or more types of cells further with liver-derived cells. The culture for obtaining the above-mentioned culture supernatant may be a co-culture.

A further aspect of the present invention is a method for culturing embryonic membrane-derived cells, comprising co-culturing liver-derived cells with one or more types of cells selected from a group consisting of intestine-derived cells, lung-derived cells, kidney-derived cells, and head-derived cells.

A further aspect of the present invention is a culture method for embryonic membrane-derived cells, comprising culturing the embryonic membrane-derived cells in the presence of a culture supernatant obtained by culturing liver-derived cells and one or more types of cells selected from a group consisting of intestine-derived cells, lung-derived cells, kidney-derived cells, and head-derived cells. The above-mentioned culture supernatant may comprise culture supernatant obtained by culturing the liver-derived cells and the one or more types of cells further with embryonic membrane-derived cells. The culture for obtaining the above-mentioned culture supernatant may be a co-culture.

A further aspect of the present invention is a method for culturing lung-derived cells, comprising co-culturing liver-derived cells and one or more types of cells selected from the group consisting of intestine-derived cells, embryonic membrane-derived cells, kidney-derived cells, and head-derived cells.

A further aspect of the present invention is a method for culturing lung-derived cells, comprising culturing the lung-derived cells in the presence of a culture supernatant obtained by culturing liver-derived cells and one or more types of cells selected from a group consisting of intestine-derived cells, embryonic membrane-derived cells, kidney-derived cells, and head-derived cells. The above-mentioned culture supernatant may comprise a culture supernatant obtained by culturing the liver-derived cells and the one or more types of cells further with lung-derived cells. The culture for obtaining the above-mentioned culture supernatant may be a co-culture.

A further aspect of the present invention is a method for culturing kidney-derived cells, comprising co-culturing liver-derived cells and one or more types of cells selected from a group consisting of embryonic membrane-derived cells and lung-derived cells.

A further aspect of the present invention is a culture method for kidney-derived cells, comprising culturing the kidney-derived cells in the presence of a culture supernatant obtained by culturing liver-derived cells and one or more types of cells selected from a group consisting of embryonic membrane-derived cells and lung-derived cells. The above-mentioned culture supernatant may comprise a culture supernatant obtained by culturing the liver-derived cells and the one or more types of cells further with kidney-derived cells. The culture for obtaining the above-mentioned culture supernatant may be a co-culture.

A further aspect of the present invention is a method for culturing intestine-derived cells, comprising co-culturing the intestine-derived cells with liver-derived cells. One or more types of cells selected from the group consisting of lung-derived cells, embryonic membrane-derived cells, head-derived cells, and kidney-derived cells may be co-cultured.

A further aspect of the present invention is a culture method for intestinal cells, comprising culturing the intestinal cells in the presence of a culture supernatant obtained by culturing liver cells. The above-mentioned culture supernatant may comprise a culture supernatant obtained by culturing the liver cells further with one or more types of cells selected from the group consisting of lung-derived cells, embryonic membrane-derived cells, head-derived cells, and kidney-derived cells. The above-mentioned culture supernatant may comprise a culture supernatant obtained by culturing the liver cells and the one or more types of cells further with intestine-derived cells. The culture for obtaining the above-mentioned culture supernatant may be co-culture.

A further aspect of the present invention is a method for culturing head-derived cells, comprising co-culturing with liver-derived cells. One or more types of cells selected from the group consisting of intestine-derived cells, lung-derived cells, and embryonic membrane-derived cells may be co-cultured.

A further aspect of the present invention is a culture method for head-derived cells, comprising culturing the head-derived cells in the presence of a culture supernatant obtained by culturing liver-derived cells. The culture supernatant may further comprise a culture supernatant obtained by culturing the liver-derived cells with one or more types of cells selected from the group consisting of intestine-derived cells, lung-derived cells, and embryonic membrane-derived cells. The culture supernatant may comprise a culture supernatant obtained by culturing the liver-derived cells and the one or more types of cells further with head-derived cells. The culture for obtaining the culture supernatant may be a co-culture.

A further aspect of the present invention is a cell culture system in which liver-derived cells and one or more types of cells selected from the group consisting of intestinal-derived cells, lung-derived cells, and embryonic membrane-derived cells are co-cultured. In addition, head-derived cells and/or kidneys may be co-cultured.

A further aspect of the present invention is a cell culture system in which embryonic membrane-derived cells, liver-derived cells, and one or more types of cells selected from the group consisting of intestine-derived cells, lung-derived cells, kidney-derived cells, and head-derived cells are co-cultured.

A further aspect of the present invention is a cell culture system in which lung-derived cells, liver-derived cells, and one or more types of cells selected from the group consisting of intestine-derived cells, embryonic membrane-derived cells, kidney-derived cells, and head-derived cells are co-cultured.

A further aspect of the present invention is a cell culture system in which kidney-derived cells, liver-derived cells, and one or more types of cells selected from a group consisting of kidney-derived cells, liver-derived cells, embryonic membrane-derived cells, and lung-derived cells are co-cultured.

A further aspect of the present invention is a cell culture system in which intestine-derived cells and liver-derived cells are co-cultured. In addition, one or more types of cells selected from the group consisting of lung-derived cells, embryonic membrane-derived cells, head-derived cells, and kidney-derived cells may be co-cultured.

A further aspect of the present invention is a cell culture system in which head-derived cells and liver-derived cells are co-cultured. In addition, one or more types of cells selected from the group consisting of intestine-derived cells, lung-derived cells, and embryonic membrane-derived cells may be co-cultured.

A further aspect of the present invention is a culture system for liver-derived cells, comprising a culture supernatant obtained by culturing one or more types of cells selected from a group consisting of intestine-derived cells, lung-derived cells, and embryonic membrane-derived cells. The culture supernatant may further comprise a culture supernatant obtained by culturing head-derived cells and/or kidney-derived cells. The culture supernatant may also comprise a culture supernatant obtained by culturing liver-derived cells. The culture for obtaining the culture supernatant may be a co-culture.

A further aspect of the present invention is a culture system for embryonic membrane-derived cells, comprising a culture supernatant obtained by culturing liver-derived cells and a culture supernatant obtained by culturing one or more types of cells selected from a group consisting of intestine-derived cells, lung-derived cells, kidney-derived cells, and head-derived cells. The above-mentioned culture supernatant may also comprise a culture supernatant obtained by culturing embryonic membrane-derived cells. The culture for obtaining the above-mentioned culture supernatant may be a co-culture.

A further aspect of the present invention is a culture system for lung-derived cells, comprising a culture supernatant obtained by culturing liver-derived cells and a culture supernatant obtained by culturing one or more types of cells selected from a group consisting of intestine-derived cells, embryonic membrane-derived cells, kidney-derived cells, and head-derived cells. The above-mentioned culture supernatant may also comprise a culture supernatant obtained by culturing lung-derived cells. The culture for obtaining the above-mentioned culture supernatant may be a co-culture.

A further aspect of the present invention is a culture system for kidney-derived cells, comprising a culture supernatant obtained by culturing liver-derived cells and a culture supernatant obtained by culturing one or more types of cells selected from a group consisting of embryonic membrane-derived cells and lung-derived cells. The above-mentioned culture supernatant may further comprise a culture supernatant obtained by culturing kidney-derived cells. The culture for obtaining the above-mentioned culture supernatant may be a co-culture.

A further aspect of the present invention is a culture system for intestine-derived cells, comprising a culture supernatant obtained by culturing liver-derived cells. The culture supernatant may further comprise a culture supernatant obtained by culturing one or more types of cells selected from the group consisting of lung-derived cells, embryonic membrane-derived cells, head-derived cells, and kidney-derived cells. The culture supernatant may further comprise a culture supernatant obtained by culturing intestine-derived cells. The culture for obtaining the culture supernatant may be co-culture.

A further aspect of the present invention is a system for culturing cells,comprising a cell culture vessel for proliferating the cells and a device for obtaining the culture supernatant contained in the medium for culturing the cells, wherein the device is a device for co-culturing multiple cell types. The device may comprise multiple cell culture vessels, and some or all of the multiple cell culture vessels may be connected so that the culture supernatant inside the vessels circulates.

### = = Cross-references to Related Literature = =

This application claims priority based on JP 2022-001828, filed on January 7, 2022, which is incorporated herein by reference.

### [Brief Description of Drawings]

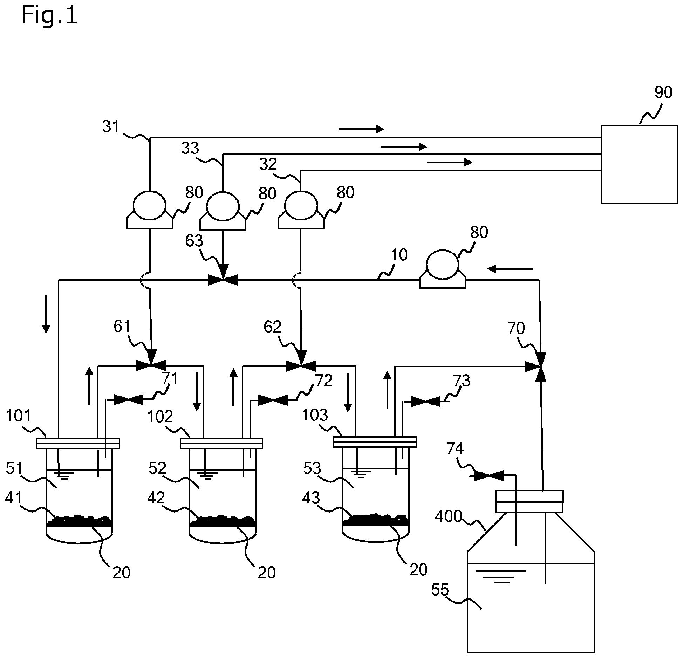

Figure 1 shows an embodiment of a culture system for obtaining culture supernatant for culturing cells to be grown.

### [Embodiments for Carrying Out the Invention]

The objects, features, advantages, and ideas of the invention are apparent to those skilled in the art from the description herein, and those skilled in the art can easily reproduce the invention. The embodiments and specific examples, etc., of the invention described below are to show preferred embodiments of the invention and are shown by way of example or illustration only, and do not limit the invention thereto. It is obvious to those skilled in the art that various changes and modifications can be made based on the description within the intent and scope of the invention disclosed herein.

### [1] Culture Method

### <Culture Method for Liver-Derived Cells>

One embodiment of the present invention is a culture method of liver-derived cells, comprising co-culturing them with one or more types of cells selected from a group consisting of intestin-derived cells, lung-derived cells, and embryonic membrane-derived cells. In addition, head-derived cells and/or kidney-derived cells may be co-cultured.

Another embodiment of the present invention is a culture method of liver-derived cell , comprising culturing them in the presence of one more more culture supernatants obtained by culturing one or more types of cells selected from a group consisting of intestin-derived cells, lung-derived cells, and embryonic membrane-derived cells. The culture supernatants may be a culture supernatant obtained by co-culturing them further with head-derived cells and/or kidney-derived cells. In any case, the culture supernatants may be a culture supernatant obtained by co-culturing them further with liver-derived cells.

### <Culture Method for Head-Derived Cells>

One embodiment of the present invention is a method of culturing head-derived cells, comprising co-culturing them with liver-derived cells and embryonic membrane-derived cells, or co-culturing them with liver-derived cells and lung-derived cells.

Another embodiment of the present invention ia a culture method of head-derived cells, comprising culturing them in the presence of a culture supernatant of liver-derived cells and a culture supernatant of embryonic membrane-derived cells, or in the presence of a culture supernatant obtained by co-culturing liver-derived cells and lung-derived cells. In any case, the culture supernatant may be a culture supernatant obtained by co-culturing them further with the head-derived cells.

### <Culture method for Intestine-Derived Cells >

One embodiment of the present invention is a culture method of intestin-derived cells, comprising co-culturing them with liver-derived cells. In addition, they may be co-cultured with one or more types of cells selected from the group consisting of lung-derived cells, embryonic membrane-derived cells, head-derived cells, and kidney-derived cells.

Another embodiment of the present invention is a culture method of intestin-derived cells, comprising culturing them in the presence of a culture supernatant obtained by culturing liver-derived cells. The culture supernatant may further contain one or more culture supernatants selected from the group consisting of culture supernatants of lung-derived cells, culture supernatants of embryonic membrane-derived cells, culture supernatants of head-derived cells, and culture supernatants of kidney-derived cells. In any case, the culture supernatant may further be a culture supernatant obtained by co-culturing them further with the intestin-derived cells.

### <Culture method of kidney-derived cells>

One embodiment of the present invention is a culture method for kidney-derived cells, comprising co-culturing them with liver-derived cells and embryonic membrane-derived cells, or co-culturing them with liver-derived cells and lung-derived cells.

Another embodiment of the present invention is a culture method of kidney-derived cells, comprising cultuing them in the presence of a culture supernatant containing a culture supernatant of liver-derived cells and a culture supernatant of embryonic membrane-derived cells, or a culture supernatant containing a culture supernatant of liver-derived cells and a culture supernatant of lung-derived cells. In any case, the culture supernatant may be a culture supernatant obtained by co-culturing them further with kidney-derived cells.

### <Culture method of lung-derived cells>

One embodiment of the present invention is a method of culturing lung-derived cells, comprising co-culturing them with one or more types of cells selected from the group consisting of liver-derived cells and head-derived cells, kidney, lung, and embryonic membrane.

Another embodiment of the present invention is a culture method of kidney-derived cells, comprising culturing them in the presence of a culture supernatant containing a culture supernatant of liver-derived cells and one or more culture supernatants selected from the group consisting of a culture supernatant of head-derived cells, a culture supernatant of kidney-derived cells, a culture supernatant of lung-derived cells, and a culture supernatant of embryonic membrane-derived cells. In any case, the culture supernatant may be the culture supernatant obtained by co-culturing them further with lung-derived cells.

### <Embryonic membrane-derived cell culture method>

One embodiment of the present invention is a culture method for embryonic membrane-derived cells, comprising co-culturing them with liver-derived cells, and one or more types of cells selected from the group consisting of head-derived cells, kidney-derived cells, lung-derived cells, and intestin-derived cells.

Another embodiment of the present invention is a culture method of embryonic membrane-derived cells, comprising culturing them in the presence of a culture supernatant containing one or more culture supernatants selected from the group consisting of a culture supernatant of liver-derived cells, a culture supernatant of head-derived cells, a culture supernatant of kidney-derived cells, a culture supernatant of lung-derived cells, and a culture supernatant of intestin-derived cells. In any case, the culture supernatant may be the culture supernatant obtained by co-culturing them further with the embryonic membrane-derived cells.

### [2] Culture system

### <Culture system for liver-derived cells>

One embodiment of the present invention is a culture system for liver-derived cells, in which liver-derived cells are co-cultured with one or more types of cells selected from a group consisting of intestin-derived cells, lung-derived cells, and embryonic membrane-derived cells. In addition, head-derived cells and/or kidney-derived cells may be co-cultured.

Another embodiment of the present invention is a culture system for liver-derived cells, in which the liver-derived cells are cultured in the presence of culture supernatant obtained by culturing one or more types of cells selected from the group consisting of intestin-derived cells, lung-derived cells, and embryonic membrane-derived cells. The culture supernatant may include a culture supernatant obtained by co-culturing them further with head-derived cells and/or kidney-derived cells. The culture supernatant may include a culture supernatant obtained by co-culturing them further with head-derived cells and/or kidney-derived cells. In any case, the culture supernatant may include culture supernatant obtained by co-culturing them further with liver-derived cells.

### <Culture System for Head-derived Cells>

One embodiment of the present invention is a culture system for head-derived cells, in which head-derived cells are co-cultured with liver-derived cells and embryonic membrane-derived cells, or co-cultured with liver-derived cells and lung-derived cells.

In another embodiment of the present invention, a culture system for head-derived cells is a culture system in which the head-derived cells are cultured with liver-derived cells and lung-derived cells or in the presence of a culture supernatant obtained by culturing liver-derived cells and embryonic membrane-derived cells. In any case, the culture supernatant may include a culture supernatant obtained by co-culturing them further with head-derived cells.

### <Intestinal-derived culture system>

One embodiment of the present invention is a culture system for intestin-derived cells, in which the intestin-derived cells are co-cultured with liver-derived cells. They may be co-culturedfurther with one or more types of cells selected from the group consisting of lung-derived cells, embryonic membrane-derived cells, head-derived cells, and kidney-derived cells.

Another embodiment of the present invention is a culture system for intestinal-derived cells, in which intestinal-derived cells are cultured in the presence of a culture supernatant obtained by culturing liver-derived cells. The culture supernatant may also contain a culture supernatant obtained by culturing one or more types of cells selected from a group consisting of lung-derived cells, embryonic membrane-derived cells, head-derived cells, and kidney-derived cells. In any case, the culture supernatant may contain culture supernatant obtained by co-culturing them further with intestinal cells.

### <Culture system for kidney-derived cells>

One embodiment of the present invention is a culture system for kidney-derived cells, in which the kidney-derived cells are co-cultured with liver-derived cells and embryonic membrane-derived cells, or co-cultured with liver-derived cells and lung-derived cells.

Another embodiment of the present invention is a culture system for kidney-derived cells, in which kidney-derived cells are cultured with liver-derived cells and lung-derived cells or in the presence of a culture supernatant obtained by culturing liver-derived cells and embryonic membrane-derived cells. In any case, the culture supernatant may include a culture supernatant obtained by co-culturing them further with kidney-derived cells.

### <Lung-derived cell culture system>

One embodiment of the present invention is a culture system for lung-derived cells, in which the lung-derived cells are co-cultured with one or more types of cells selected from a group consisting of liver-derived cells, and head-derived cells, kidney, lung, and embryonic membrane.

Another embodiment of the present invention is a culture system for lung-derived cells is a culture system in which the lung-derived cells are cultured with lung-derived cells and one or more types of cells selected from a group consisting of liver-derived cells, head-derived cells, kidney-derived cells, lung-derived cells, and embryonic membrane-derived cells or in the presence of a culture supernatant obtained by culturing lung-derived cells and one or more types of cells selected from a group consisting of liver-derived cells, head-derived cells, kidney-derived cells, lung-derived cells, and embryonic membrane-derived cells. In any case, the culture supernatant may include a culture supernatant obtained by co-culturing them further with lung-derived cells.

### <Embryonic Membrane-derived Cell Culture System>

One embodiment of the present invention is a culture system for embryonic membrane-derived cells, in which embryonic membrane-derived cells are co-cultured with liver-derived cells and one or more types of cells selected from the group consisting of head-derived cells, kidney-derived cells, lung-derived cells, and lung-derived cells.

Another embodiment of the present invention is a culture system for embryonic membrane-derived cells, in which embryonic membrane-derived cells are cultured in the presence of culture supernatant obtained by co-culturing liver-derived cells and one or more types of cells selected from the group consisting of head-derived cells, kidney-derived cells, lung-derived cells, and lung-derived cells. In any case, the culture supernatant may include a culture supernatant obtained by co-culturing them further with the embryonic membrane-derived cells.

### <Specific culture system>

The term "culture system" refers to a system that is in a state of culture, and includes a culture device including a cell culture vessel, a cell culture medium, and the cultured cells. The culture system may include a medium supply container that supplies a fresh cell culture medium to the culture system, and a medium recovery container that collects the cell culture medium after it has been used. The cell culture vessel is not particularly limited and, for example, it may be a culture tank, a glass petri dish, or a plastic dish. The cell culture medium can be selected by the person skilled in the art to be suitable for the cells to be cultured. The medium may be a serum-containing medium, but a serum-free medium is preferable in that the substances contained in the medium can be easily controlled.

In the case of co-culture, multiple types of cells may be cultured in a single culture vessel, but the cells may also be grown separately by providing independent culture vessels for each cell type and sharing the culture medium. In the latter case, the culture vessels may be joined together so that the culture medium is connected between each culture vessel. The method of joining the culture vessels together can be determined as appropriate for the purpose. For example, some or all of the culture vessels, including the culture vessel for the target cells to be grown, may be joined together in a ring shape. Alternatively, the culture vessel for the target cells to be grown may be joined independently to each of the culture vessels for the cells to be co-cultured.

When culturing the target cells to be grown by adding culture supernatant, the culture vessel for the target cells may be independent of vessels for the cells used to obtain the culture supernatant. The number of the vessels for the cells used to obtain the culture supernatant may be one or more, regardless of the number of cell types used, but it is preferable to have one or more for each cell type. They may be independent or connected according to the purpose. It is preferable to include cells of the same type as the target cells for the cells for obtaining the culture supernatant.

The tubes used as connecting tubes to join the culture vessels together may be equipped with a control device such as a valve to control opening and closing, and the medium may be controlled by controlling the opening and closing. In addition, a pump may be provided on the tube between the culture vessels to flow the medium in either direction.

For example, when there are two culture vessels, they may be connected by a single tube, or by two or more tubes. By connecting the culture vessels with multiple tubes, the culture medium can be recirculated. When there are three or more culture vessels, they may be connected in series or in parallel, and the culture vessels may be connected as appropriate to suit the purpose, to form an appropriate culture system.

There may be one or more than one culture vessels for each cell type. In either case, the culture vessels can be combined as appropriate to suit the purpose, to form an appropriate culture system.

The specific methods for preparing and culturing cells are explained below.

### [3] Specific cell preparation and culture methods

### <Cell Source>

The animals from which the cells are obtained are not particularly limited, but vertebrates such as mammals are preferable, and the animals that lay eggs with egg shells, such as birds and reptiles are more preferable. In particular, the species from which liver-derived cells, head-derived cells, kidney-derived cells, or lung-derived cells originate are not particularly limited, but vertebrates are preferable, and they may be mammals, reptiles, birds, or amphibians, for example, humans, cows, pigs, rabbits, chickens, sheep, goats, etc. In addition, the species from which the embryonic membrane-derived cells originate is not particularly limited if they are oviparous, and may be birds or reptiles, or even chickens. In the case of chickens, they may be 0-day to 21-day embryos, butmay be 2-day to 19-day embryos, 4-day to 17-day embryos, or 6-day to 15-day embryos. Furthermore, cells obtained from each tissue may be derived from the same animal or from different animals.

### <Types of cells and preparation methods>

Liver-derived cells, head-derived cells, intestine-derived cells, kidney-derived cells, and lung-derived cells can be obtained by collecting the liver, head which is the part above the neck and may or may not include the neck, intestines, kidneys, and lungs, respectively, from an animal embryo and separating cells by chemical processing using enzymes such as collagenase, dispase, and trypsin, or by physical processing such as cutting and crushing with a scalpel or razor blade, or pipetting using a microchip. The specific methods have already been established as common technical knowledge, and there are no particular limitations as long as the cells can be recovered alive by the method.

Liver-derived cells include one or more types of cells selected from the group consisting of parenchymal cells (i.e., hepatocytes), non-parenchymal cells (e.g., sinusoidal wall cells, myofibroblasts, bile duct epithelial cells, connective tissue cells, hepatic stellate cells, etc.), and their progenitor or stem cells.

Head-derived cells include one or more types of cells selected from the group consisting of retinal cells, neuronal cells, glial cells, pituitary cells, muscle cells, connective tissue cells, fibroblasts, vascular endothelial cells, vascular endothelial cells, and their progenitor or stem cells.

Intestin-derived cells include one or more types of cells selected from the group consisting of intestinal epithelial cells, CBC cells, Paneth cells, enteroendocrine cells, goblet cells, absorptive epithelial cells, M cells, intestinal wall cells, connective tissue cells, muscle cells, fibroblasts, vascular endothelial cells, vascular endothelial cells, and their progenitor or stem cells. The intestine may be the duodenum, small intestine, large intestine, or colon, or a mixture of any two or more of these.

Kidney-derived cells include one or more types of cells selected from the group consisting of glomerulus-constituting epithelial cells, endothelial cells, mesangial cells, visceral epithelial cells, and parietal visceral epithelial cells; renal tubule-constituting endothelial cells and epithelial cells; and connective tissue cells filling in other tissues; fibroblasts; vascular endothelial cells; vascular endothelial cells; and their progenitor or stem cells.

Lung-derived cells include one or more types of cells selected from the group consisting of alveolar macrophages, type II alveolar epithelial cells, type I alveolar epithelial cells, basal cells, ciliated cells, goblet cells, mucous cells, serous cells, connective tissue cells, myocytes, fibroblasts, vascular endothelial/endothelial cells, and their progenitor or stem cells.

Embryonic membrane-derived cells include one or more types of cells selected from the group consisting of amnion, serous membrane, allantois, and yolk sac.

The cells may be primary cultured cells or established cell lines. They may also be cultured in the form of tissues or organs, in the form of cell masses, or as individually isolated cells.

Cultivation may be suspension culture or adhesion culture. It may also be planar culture or 3D culture. The choice of which type of culture to use can be easily and appropriately determined by a person skilled in the art, depending on the cell type.

### <Specific cell culture methods>

The specific cell culture method allows the person skilled in the art to select a method suitable for the cells to be cultured using the culture system described above.

In the case of co-culture, it is possible to mix and culture multiple types of cells, but it is also possible to grow the cells separately by dividing the culture vessel into compartments for each cell type and sharing the culture medium. Alternatively, it is possible to prepare individual vessels for each cell type and join the vessels together so that the medium is shared between them and becomes a uniform medium throughout. Alternatively, individual vessels may be prepared for each cell and the vessels are joined together so that the medium is connected, but the flow of the medium is controlled so that the medium is connected between the vessels for some period and the medium stays within each vessel without being connected for other period.

When culture supernatants of other cell types are used for culturing, the culture supernatant can be collected after culturing the cells at a density of 1 × 10⁴ to 1 × 10⁷ cells/mL in a cell vessel for 1 to 30 days, for example. The supernatant can then be centrifuged to remove any cell residue or other precipitates. The resulting culture supernatant may be used as is (i.e. 100% cell supernatant) to culture the cells intended for proliferation, but it may also be used with fresh medium added at a rate of 10% or more, 30% or more, 50% or more, or 70% or more may be used. When adding the culture supernatant of two or more cell types to the medium for the cells to be grown, the two or more cell types used to obtain the culture supernatant may be cultured separately and then the supernatants mixed, or some or all of the cells may be co-cultured. When co-culturing, some or all of the co-cultured cells may be cultured in a single culture vessel, but the cells may also be grown separately by providing separate culture vessels for each cell type and culturing them in a shared medium. In the latter case, the culture vessels may be joined together so that the medium is connected between each culture vessel. The method of joining the culture vessels together can be decided as appropriate for the purpose. For example, some or all of the culture vessels can be joined together in a ring shape, and the culture vessels for cells of the same type as the target cells for growth can be included.

### <Culture system for obtaining culture supernatant>

An example of a culture system for obtaining culture supernatant using a culture device that includes culture vessels and tubes for connecting the vessels is shown below referring to Figure 1. However, the embodiment of the culture system is not limited thereto, and a person skilled in the art would be able to easily make various improvements and modifications.

This culture system has multiple culture vessels 101-103, including scaffolds 20 for cell culture, cultured cells 41-43, and culture supernatants 51-53. The cultured cells 41-43 cultured in each culture vessel 101-103 may be the same type or different types, and may include cell types to be grown using the obtained supernatant. The culture vessels 101-103 are connected by a connecting tube 10 so that the culture supernatant 51-53 can be circulated, and three-way valves 61-63 are provided between each of the vessels 101-103. Collection tubes 31-33 are provided so that the supernatants can be collected in a collection vessel 90 by controlling each of the three-way valves 61-63 from this culture medium circulation system. In addition, a medium container 400 containing a fresh medium 55 is connected to this medium circulation system using a three-way valve 70. Pumping devices 80 are provided on the connecting tube 10 and the collection tubes 31-33. In addition, the culture vessels 101-103 and the medium container 400 are equipped with pipes that open to the outside air, with opening/closing valves 71-73 and 74, and the pipes are equipped with filters to prevent microorganisms and other small particles from entering from the outside air.

Using such a device, it is possible to collect the supernatants that match the desired purpose. For example, by opening valve 61 only in the direction from the culture vessel 101 to the collection vessel 90, and opening valves 63 and 70 only in the direction from the medium vessel 400 to the culture vessel 101, it is possible to obtain the culture supernatant in which only the cultured cells 41 are cultured. Alternatively, by opening the valves 61 to 63 and 70 only such that the medium circulates, a uniformed culture supernatant in which the cultured cells 41 to 43 are co-cultured circulates through the circulation system. After that, by opening the valve 71 and opening the valve 63 only in the direction from the vessel 103 to the collection container 90, the uniformed culture supernatant can be collected. After that, by closing the valve 71, opening the valves 73 and 74, and opening the valves 61 to 63 in the direction that allows the medium to circulate, and opening the valve 70 in the direction from the medium container 400 to the culture vessel 101, a fresh medium is supplied to the culture vessels 101 to 103. Alternatively, by appropriately combining the opening and closing of each valve, it is possible to freely control the amount of medium in each of the culture vessels 101-103, as well as the combination of medium to be recovered, etc.

In this way, the supernatants that match the object can be collected in the collection container 90, and the collected supernatants can be used to culture cells to be grown. In addition, the collection container 90 and the culture vessel for cells to be grown may be connected, and the supernatants collected in the collection container 90 may be supplied automatically to the culture vessel for cells to ne grown. In this case, the amount of supernatants supplied, the frequency of supply, the number of times it is supplied, etc., may be computer-controlled.

### [Examples]

### [Example 1]

### <Cell Preparation Method>

Cells were prepared from each tissue as follows.

First, fertilized chicken eggs were incubated, and mixed membranes (referred to herein as 3Mix) of the embryonic membrane, allantois, and yolk membrane, head, liver, kidney, lung, and intestine were each collected from 12-day embryos, and the tissues were dispersed into single cells by chemical treatment with collagenase or physical treatment using a mesh (100 µm).

The collected cells were seeded at a density of 1 × 108 cells in a 500 ml culture bottle (BelloCell: CESCO Co., Ltd.) and cultured for 2 weeks. The medium used was DMEM supplemented with 10% FBS (fetal bovine serum).

### <Cell culture method>

### (1) Preparation of culture supernatant for each cell combination

Using the above culture medium, the cells were seeded at a density of 1 × 10⁸ or more cells per 500 ml culture bottle, and then cultured at 37°C in the presence of 5% CO₂ using a BioNOCC II matrix (CESCO Bioengineering). Two weeks after the start of culture, the supernatant from each culture was collected, centrifuged to remove cell residue and other precipitates, and the resulting supernatants were used in the following culture.

### (2) Culturing

The cells to be used for the proliferation experiment were seeded at a density of at least 5 × 10⁵ cells/ml in plastic dishes for cell culture together with cell scaffolds, and cultured at 37°C for 10 days in the presence of 5% CO₂. The culture medium used at the start of the culture was a basic food culture medium, to which 50% of the culture supernatant of the other cell types obtained in (1) was added. During days 0-6 of the culture, the medium was replaced 50% every 2 days. During days 6-10 of the culture, the medium was replaced 50% every day. For the medium replacement, the above medium without the culture supernatant was used. After 10 days of culture, the cells were collected, the number of cells was counted, and the value when the number of cells at the time of seeding was set to 1 was calculated, and the results are shown in Result I of Table 1,. The experiment was conducted using three dishes for each, and the average values are listed in Table 1. The growth rate when cultured alone is set to 1 and the growth rates were calculated. The results are listed in Result II of Table 1,.

**[Table 1]**

| | Cultured cells | Supernatant | | Results | |
|---|---|---|---|---|---|
| | | | | I | II |
| 1 | Liver | - | - | 0.3 | 1.0 |
| 2 | | 3Mix | - | 1.0 | 3.3 |
| 3 | | Lung | - | 1.0 | 3.3 |
| 4 | | Kidney | - | 0.3 | 1.0 |
| 5 | | Intestine | - | 0.5 | 1.7 |
| 6 | | Head | - | 0.3 | 1.0 |
| 7 | | 3Mix | Lung | 1.9 | 6.3 |
| 8 | | 3Mix | Kidney | 1.3 | 4.3 |
| 9 | | 3Mix | Intestine | 0.8 | 2.7 |
| 10 | | 3Mix | Head | 2.1 | 7.0 |
| 11 | | Lung | Kidney | 2.3 | 7.7 |
| 12 | | Lung | Intestine | 1.8 | 6.0 |
| 13 | | Lung | Head | 18.0 | 60.0 |
| 14 | | Kidney | Intestine | 1.3 | 4.3 |
| 15 | | Kidney | Head | 0.4 | 1.3 |
| 16 | | Intestine | Head | 0.9 | 3.0 |
| 17 | 3Mix | - | - | 1.9 | 1.0 |
| 18 | | Liver | - | 1.7 | 0.9 |
| 19 | | Liver | Lung | 3.0 | 1.6 |
| 20 | | Liver | Kidney | 2.4 | 1.3 |
| 21 | | Liver | Intestine | 3.0 | 1.6 |
| 22 | | Liver | Head | 2.9 | 1.5 |
| 23 | Lung | - | - | 1.4 | 1.0 |
| 24 | | Liver | - | 1.4 | 1.0 |
| 25 | | Liver | 3Mix | 2.2 | 1.6 |
| 26 | | Liver | Kidney | 3.7 | 2.6 |
| 27 | | Liver | Intestine | 8.3 | 5.9 |
| 28 | | Liver | Head | 2.3 | 1.6 |
| 29 | Kidney | - | - | 1.0 | 1.0 |
| 30 | | Liver | - | 0.4 | 0.4 |
| 31 | | Liver | 3Mix | 2.5 | 2.5 |
| 32 | | Liver | Lung | 3.7 | 3.7 |
| 33 | | Liver | Intestine | 0.3 | 0.3 |
| 34 | | Liver | Head | 0.6 | 0.6 |
| 35 | Intestine | - | - | 2.2 | 1.0 |
| 36 | | Liver | - | 3.6 | 1.6 |
| 37 | | Liver | 3Mix | 8.5 | 3.0 |
| 38 | | Liver | Lung | 8.3 | 3.8 |
| 39 | | Liver | Kidney | 7.2 | 3.3 |
| 40 | | Liver | Head | 5.9 | 2.7 |
| 41 | Head | - | - | 0.4 | 1.0 |
| 42 | | Liver | - | 0.3 | 0.8 |
| 43 | | Liver | 3Mix | 1.7 | 4.3 |
| 44 | | Liver | Lung | 2.3 | 5.8 |
| 45 | | Liver | Kidney | 0.4 | 1.0 |
| 46 | | Liver | Intestine | 0.6 | 1.5 |

### <Result Analysis>

If the growth of cells is promoted by adding the culture supernatant of other cells to the culture medium, compared to the value when each cell is cultured alone, the value in Result II should be greater than 1.0. For liver cells, the supernatant of 3Mix, lung, or intestinal cells, and the mixed supernatant of head cells and kidney cells; for 3Mix cells, the mixed supernatant of liver cells and lung, intestinal, kidney, or head cells supernatant; for lung cells, a mixture of the supernatant of liver cells and the supernatant of 3Mix, intestine, kidney or head cells; for kidney cells, a mixture of the supernatant of liver cells and the supernatant of 3Mix or lung cells supernatant; and for intestinal cells, the supernatant of liver cells, and for head cells, the supernatant of liver cells, preferably the supernatant of liver cells and the supernatant of 3Mix, lung, or intestinal cells, could promote cell growth.

### [Example 2]

### <Preparation of Cells>

Cells were prepared from each tissue as follows.

First, fertilized chicken eggs were incubated, and 3 Mixture, liver, and lung were each collected from 12-day-old embryos, and the tissues were dispersed by physical treatment using scissors or a mesh (100 µm).

### <Culture method using a reflux culture system>

### (1) Preparation of culture supernatants for each cell combination

The reflux culture method is performed by the constitution of a culture system in which a diaphragm-type quantitative pump (SIMDOS Co., Ltd.) was used to circulate a single 500 ml bottle of culture medium and three 500 ml bottles of culture medium containing BioNOCCIi three 500 ml culture bottles containing a 500 ml culture bottle with a diaphragm-type quantitative feed pump (SIMDOS Co., Ltd.) and a BiONOCII Using the same basic culture medium for food as in Example 1, 3Mix, liver, and lung cells were seeded at 1 × 10⁸ cells each in separate culture bottles and cultured at 37°C in the presence of 5% CO₂. After 12 days of incubation, the culture supernatants were collected, centrifuged to remove cell debris and other precipitates, and the resulting culture supernatants were used in the following experiments.

### (2) Cultivation

The cells (derived from duck liver) to be used to investigate proliferation were seeded at a density of 2 × 10⁴ cells/ml in plastic plates for cell culture and cultured at 37°C for 2 days in the presence of 5% CO₂. The culture medium used at the start of the culture was a basic culture medium for food use, to which 50% of the culture supernatant obtained in (1) was added. On the second day of incubation, we used the CellTiter-Glo 2.0 Cell viability assay kit (Promega) to measure the amount of ATP as an indicator of cell count. The cell number when cultured in food medium alone was set to 1 and the values for the cell numbers were caliculated. For each experiment, we used three wells and calculated the average value, which yielded a value of 5.0 to 24.4.

Even when co-culturing multiple cells to obtain the supernatant and using the resulting supernatant, an effect of growing liver-derived cells is observed.

### [Industrial applicability]

This invention provides a culture method of liver-derived cells and a culture system including liver-derived cells.

## Claims

1. A culture method of liver-derived cells, comprising the step of co-culturing the liver-derived cells with one or more types of cells selected from a group consisting of intestin-derived cells, lung-derived cells, and embryonic membrane-derived cells.

2. The culture method of Claim 1, wherein head-derived cells and/or kidney-derived cells are further co-cultured.

3. A culture method for liver-derived cells, comprising the step of culturing the liver-derived cells in the presence of a culture supernatant obtained by culturing one or more types of cells selected from the group consisting of intestin-derived cells, lung-derived cells, and embryonic membrane-derived cells.

4. The culture method of Claim 3, wherein the culture supernatant comprises a culture supernatant obtained by culturing the one or more types of cells further with head-derived cells and/or kidney-derived cells.

5. The culture method of Claim 3 or 4, wherein the culture supernatant comprises a culture supernatant obtained by culturing the one or more types of cells further with liver-derived cells.

6. The culture method of any one of Claims 3 to 5, wherein the culture is a co-culture.

7. The culture method of any one of Claims 1 to 6, wherein the liver-derived cells are one or more types of cells selected from the group consisting of parenchymal cells (hepatocytes), non-parenchymal cells (sinusoidal wall cells, myofibroblasts, bile duct epithelial cells, connective tissue cells, and hepatic stellate cells), and progenitor cells or stem cells thereof.

8. The culture method of any one of Claims 1 to 6, wherein the intestine-derived cells are one or more types of cells selected from the group consisting of intestinal epithelial cells, CBC cells, Paneth cells, enteroendocrine cells, goblet cells, absorptive epithelial cells, M cells, intestinal wall cells, connective tissue cells, muscle cells, fibroblasts, vascular endothelial/pericyte cells, and progenitor cells or stem cells thereof.

9. The culture method of any one of Claims 1 to 6, wherein the lung-derived cells are one or more types of cells selected from the group consisting of alveolar macrophages, type II alveolar epithelial cells, type I alveolar epithelial cells, basal cells, ciliated cells, goblet cells, mucous cells, serous cells, connective tissue cells, muscle cells, fibroblasts, endothelial/epithelial cells of blood vessels, and progenitor cells or stem cells thereof.

10. The culture method of any one of Claims 1 to 6, wherein the embryonic membrane-derived cells are one or more types of cells selected from the group consisting of amnion, serous membrane, allantois, and yolk sac.

11. The culture method of Claim 2 or 4, wherein the head-derived cells are one or more types of cells selected from the group consisting of retinal cells, neuronal cells, glial cells, pituitary cells, muscle cells, connective tissue cells, fibroblasts, vascular endothelial/endothelial cells, and progenitor cells or stem cells thereof.

12. The culture method of Claim 2 or 4, wherein the kidney-derived cells are one or more types of cells selected from the group consisting of the glomerulus-constituting epithelial cells, endothelial cells, mesangial cells, visceral epithelial cells, and parietal visceral cells; tubule-constituting endothelial cells and epithelial cells; connective tissue cells filling in other tissues; fibroblasts; endothelial/endothelial cells; and progenitor cells or stem cells thereof.

13. The culture method of any one of Claims 1 to 6, wherein the cells are cultured using a serum-free medium.

14. A culture system for cells wherein
liver-derived cells, and
one or more types of cells selected from the group consisting of intestin-derived cells, lung-derived cells, and embryonic membrane-derived cells,
are co-cultured.

15. The culture system of Claim 14,wherein head-derived cells and/or kidney-derived cells are further co-cultured.

16. A culture system for liver-derived cells, comprising
a culture supernatant obtained by culturing one or more types of cells selected from a group consisting of intestin-derived cells, lung-derived cells, and embryonic membrane-derived cells.

17. The culture system of claim 16 comprising a culture supernatant obtained by culturing head-derived cells and/or kidney-derived cells.

18. The culture system of claim 16 or 17 further comprising a culture supernatant obtained by culturing liver-derived cells.

19. The culture system of any one of claims 16 to 18, wherein the culture is a co-culture.

20. The culture system of any one of claims 16 to 19, wherein the liver-derived cells are one or more types of cells selected from the group consisting of parenchymal cells (hepatocytes), non-parenchymal cells (sinusoidal wall cells, myofibroblasts, bile duct epithelial cells, connective tissue cells, and hepatic stellate cells), and progenitor cells or stem cells thereof.

21. The culture system of any one of claims 16 to 19, wherein the intestin-derived cells are one or more types of cells selected from the group consisting of intestinal epithelial cells, CBC cells, Paneth cells, enteroendocrine cells, goblet cells, absorptive epithelial cells, M cells, intestinal wall cells, connective tissue cells, muscle cells, fibroblasts, vascular endothelial/pericyte cells, and progenitor cells or stem cells thereof.

22. The culture system of any one of claims 16 to 19, wherein the lung-derived cells are one or more types of cells selected from the group consisting of alveolar macrophages, type II alveolar epithelial cells, type I alveolar epithelial cells, basal cells, ciliated cells, goblet cells, mucous cells, serous cells, connective tissue cells, myocytes, fibroblasts, endothelial/epithelial cells of blood vessels, and progenitor cells or stem cells thereof.

23. The culture system of any one of claims 16 to 19, wherein the embryonic membrane-derived cells are one or more types of cells selected from the group consisting of amnion, serous membrane, allantois, and yolk sac.

24. The culture system of claim 17 or 19, wherein the head-derived cells are one or more types of cells selected from the group consisting of retinal cells, neuronal cells, glial cells, pituitary cells, myocytes, connective tissue cells, fibroblasts, endothelial/epithelial cells, and progenitor cells or stem cells thereof.

25. The culture system of claim 17 or 19, wherein the kidney-derived cells are one or more types of cells selected from the group consisting of the glomerulus-constituting epithelial cells, endothelial cells, mesangial cells, visceral epithelial cells, and parietal visceral cells; tubule-constituting endothelial cells and epithelial cells; connective tissue cells filling in other tissues; fibroblasts; endothelial/endothelial cells; and progenitor cells or stem cells thereof.

26. The culture system of any one of claims 16 to 19, comprising a serum-free medium.
